# EUROPEAN PATENT APPLICATION

(11) **EP 1 626 087 A1**
(43) Date of publication of application: **15.02.2006**
(21) Application number: 04733092.3
(22) Date of filing: 14.05.2004
(51) Int. Cl.: C12N 15/52, C12N 1/19, C12P 7/62, C12R 1/72

(54) **IMPROVED TRANSFORMANT AND PROCESS FOR PRODUCING POLYESTER USING THE SAME**

(30) Priority: 15.05.2003 JP 2003137492
(71) Applicant: KANEKA CORPORATION, Osaka-shi, Osaka 530-8288 (JP)
(72) Inventor: OKUBO, Yuji, Takasago-shi, Hyogo 6760026 (JP); NAGAOKA, Tetsuya, Kobe-shi, Hyogo 6512124 (JP); YOKOMIZO, Satoru, Akashi-shi, Hyogo 6740092 (JP); MATSUMOTO, Keiji, NIshinomiya-shi, Hyogo 6638023 (JP); TAKAGI, Masamichi, Fuchu-shi, Tokyo 1830051 (JP); OHTA, Akinori, Saitama-shi, Saitama 3310063 (JP)
(74) Representative: HOFFMANN EITLE
(86) International application number: PCT/JP2004/006542
(87) International publication number: WO 2004/101796

(57) **Abstract**

This invention relates to provide a gene expression cassette, which comprises a gene coding for an Aeromonas caviae-derived polyhydroxyalkanoic acid (PHA) synthase wherein a mutation has been introduced, and a promoter and a terminator both capable of functioning in yeast; a transformant wherein said gene expression cassette has been introduced in yeast, and a method for producing polyester by using said transformant.

This invention enabled a production of copolyesters resulting from copolymerization of a 3-hydroxyalkanoic acid(s) and having biodegradability and good physical properties in yeast efficiently.

## Description

### TECHNICAL FIELD

The present invention relates to genes necessary for the enzymatic synthesis of copolyesters, a microorganism fermentatively synthesizing polyesters utilizing the gene, and a process for producing polyesters using the microorganism.

### BACKGROUND ART

At present, many kinds of microorganisms are known to accumulate polyesters such as polyhydroxyalkanoates (hereinafter referred to briefly as PHA) as the energy storage materials within cells. A representative example of the polyester is poly-3-hydroxybutyric acid (hereinafter referred to briefly as P(3HB)), which is a homopolymer of 3-hydroxybutyric acid (hereinafter referred to as 3HB for short). It was first discovered in Bacillus megaterium (M. Lemoigne, Ann. Inst. Pasteur, 39, 144 (1925)). P(3BH) is a thermoplastic polymer and is biodegradable in the natural environment and, thus, has recently attracted attention as an ecofriendly plastic. However, P(3HB) is high in crystallinity, and stiff and brittle material, so that the range of practical application thereof is limited. Therefore, research works have been undertaken to improve these properties.

Among others, a technology of producing a copolymer made of 3-hydroxybutyric acid (3HB) and 3-hydroxyvaleric acid (hereinafter referred to briefly as 3HV) (hereinafter such copolymer is referred to as P(3HB-co-3HV)) is disclosed (Japanese Kokai Publication Sho-57-150393 and Japanese Kokai Publication Sho-59-220192). This P(3HB-co-3HV) is rich in flexibility as compared with P(3HB), hence was expected to have a broader application range. In actuality, however, P(3HB-co-3HV) shows only slight changes in physical properties even when the molar fraction of 3HV is increased. In particular, the flexibility can not be improved to the amount required for use in films and the like. Thus, it has been used only in the field of rigid shaped articles such as shampoo bottles and disposable razor grips.

In recent years, studies have been made concerning the copolymer consisting of two components 3HB and 3-hydroxyhexanoic acid (hereinafter referred to as 3HH for short) (hereinafter such copolyesters are referred to as P(3HB-co-3HH) for short) and the technology of producing it (Japanese Kokai Publication Hei-05-93049 and Japanese Kokai Publication Hei-07-265065). According to these patent documents, this technology of producing P(3HB-co-3HH) comprises fermentative production thereof from fatty acids, such as oleic acid, or oils or fats, such as olive oil, using Aeromonas caviae isolated from soil. Studies concerning the properties of P(3HB-co-3HH) have also been made (Y. Doi, S. Kitamura, H. Abe, Macromolecules, 28, 4822-4823 (1995)). According to this report, when A. caviae is cultured using fatty acids of not less than 12 carbon atoms as the only carbon source, P(3HB-co-3HH) with a 3HH fraction of 11 to 19 mole percent can be fermentatively produced. It has been revealed that the properties of such P(3HB-co-3HH) change from hard and brittle gradually to soft and flexible, to an extent exceeding the flexibility of P(3HB-co-3HV), with the increase in molar fraction of 3HH. However, the above method of production is low in productivity, namely the cell productivity is 4 g/L and the polymer content is 30%. Therefore, methods capable of attaining higher productivity for practical use have been searched for.

A PHA synthase gene has been cloned from Aeromonas caviae, which is a producible strain of P(3HB-co-3HH) (Japanese Kokai Publication Hei-10-108682; T. Fukui, Y. Doi, J. Bacteriol., vol. 179, No. 15, 4821-4830 (1997)). This gene was introduced into Ralstonia eutropha (formerly Alcaligenes eutrophus), and cultivation was carried out using the resulting' transformant and a vegetable oil as the carbon source, whereby a content in cells of 4 g/L and a polymer content of 80% were attained (T. Fukui et al., Appl. Microbiol. Biotechnol., 49, 333 (1998)). A method of producing P(3HB-co-3HH) using bacteria, such as Escherichia coli, or a plant as the host has also been disclosed, without describing any productivity data, however (WO 00/43525, for example).

The above-mentioned polymer P(3HB-co-3HH) can be given a wide range of physical properties, from properties of rigid polymers to properties of flexible polymers, by changing the molar fraction of 3HH and therefore can be expected to be applicable in a wide range, from television boxes and the like, for which rigidity is required, to yarns, films and the like, for which flexibility is required. However, the production methods mentioned above are still poor in the productivity of P(3HB-co-3HH). There is no other way but to say that they are still unsatisfactory as practical production methods of P(3HB-co-3HH).

Recently, Kichise et al. reported a method, comprising modifying A. caviae-derived P(3HB-co-3HH) synthase by in vitro enzyme evolution system and then introducing it into Escherichia coli, to construct an Escherichia coli capable of accumulating more amount of P(3HB-co-3HH) than a wild-type gene-introduced Escherichia coli (T. Kichise et al., Appl. Environ. Microbiol. 68, 2411-2419 (2002)). In the report, E2-50 strain and T3-11 strain, in which specific activities of P(3HB-co-3HH) synthase were enhanced, were obtained. E2-50 is a mutant of P(3HB-co-3HH) synthase, in which serine is substituted for 149th amino acid asparagine, and T3-11 is one, in which glycine is substituted for 171st aspartic acid. Specific activity of the mutated enzyme in E2-50 strain is enhanced to about 1.5 times as high as that of wild type Escherichia coli, whereas that of T3-11 strain is enhanced to about 1.2 times. But in general, Escherichia coli cannot utilize fats and oils, which are low-cost carbon sources. Furthermore, it does not contain condensing enzymes of acetyl-CoA, which is necessary for a route to synthesize a component unit of P(3HB-co-3HH). Therefore, a low-cost and efficient production system cannot be attained unless other genes providing fats and oils-decomposing ability or substrate-supplying ability are otherwise introduced. Furthermore, Amara et al. obtained a mutant the specific activity of which increased fivefold, but polymer productivity was only slightly increased in the case where glucose was used as a carbon source (Amara AA. et al., Appl. Microbiol. Biotechnol. 59, 477-482 (2002)). In Escherichia coli, a mutated polymer synthase with increased activity cannot express its own abilities, and Escherichia coli cannot directly utilize vegetable oils and fats, which are low-cost carbon sources. Therefore, Escherichia coli lacks usefulness as a host for polymer production in economical viewpoint.

In a study of the production of biodegradable polyesters, Leaf et al. used yeast high in cell productivity as the host (Microbiology, vol. 142, pp. 1169-1180 (1996)). Thus, the polyester synthase gene of Ralstonia eutropha was introduced into Saccharomyces cerevisiae, a kind of yeast, the thus-produced transformant was cultured using glucose as the carbon source, and the accumulation of P(3HB) was confirmed. However, polymer content achieved in this study resulted in as low as 0.5% and the polymer was stiff and brittle P(3HB). Other studies also have been made on the production of copolymers containing a monomer unit having 5 or more carbon atoms, by expressing, in yeast Saccharomyces cerevisiae, a polyester synthase gene derived from Pseudomonas aeruginosa in the presence of fatty acids as a carbon source. But the polymer content achieved also resulted in as low as 0.5% in this case (Poirier Y. et al., Appl. Microbiol. Biotechnol. 67, 5254-5260 (2001)). Furthermore, other studies have been made on the production of polyester using oleic acid as a carbon source, by expressing and targeting, into peroxisomes of yeast Pichia Pastoris, a polyester synthase gene derived from the same Pseudomonas species. These studies showed that 1% weight of polymer was accumulated per dried cell unit (Poirier Y. et al., FEMS Microbiology Lett., vol. 207, pp.97-102 (2002)). But such low accumulation content is quite insufficient.

Yeast is known to grow fast and be high in cell productivity. Among them, yeasts belonging to the genus Candida attracted attention as single cell proteins in the past and, since then, studies have been made on the production of cells thereof for use as feeds using normal-paraffins as carbon sources. Further, in recent years, host-vector systems for the genus Candida have been developed, and the production of substances using the recombinant DNA technology has been reported (Kagaku to Seibutsu (Chemistry and Living organisms), vol. 38, No. 9, 614 (2000). When Candida utilis is used as the host, the α-amylase productivity is as high as about 12.3 g/L. Microorganisms of the genus Candida having such high substance productivity are expected to serve as hosts for polymer production. Furthermore, cells thereof can be separated from the culture fluid with ease as compared with bacteria and, thus, the polymer extraction and purification steps can be facilitated.

Thus, a method of producing P(3HB-co-3HH) having good physical properties using yeast belonging to the genus Candida, and the like, has been developed, but further improvement has been requested for polymer productivity (WO 01/88144). As one method for increasing polymer productivity per cell, there may be mentioned a method comprising increasing the amount of the polymer synthase in a cell. As examples of methods for increasing the amount of polymer synthase in a cell, there may be mentioned a method comprising using a strong promoter, a method comprising using a high-copy-number plasmid, a method comprising introducing a large number of enzyme expression units into a plasmid or chromosomes, or the like method. It has been known, however, that increase of the molecular number of enzymes per cell leads to decrease in the molecular weight of the product polymer (Sim S. J. et al., Nature Biotechnology, vol. 15, pp. 63-67 (1997); and Gerngross T.U., Martin D.P., Proc. Natl. Acad. Sci. U.S.A, vol. 92, pp. 6279-6283 (1995)). Because the molecular weight of the polymer remarkably affects properties of a biodegradable polymer, methods, which are able to increase productivity without decrease of molecular weight, are longed for.

### SUMMARY OF THE INVENTION

In view of the above-mentioned state of the art, it is an object of the present invention to provide a gene coding for a mutated PHA synthase which can be expressed in yeast in a functional and efficient manner, a yeast transformant transformed by a gene expression cassette comprising the gene, and a method for producing polyesters such as P(3HB-co-3HH) having biodegradability and good physical properties which comprises cultivating the transformant obtained in the above manner.

The present inventors made various investigations and, as a result, found that when genes, which code for amino acid sequences of a mutated Aeromonas caviae-derived PHA synthase wherein a specific mutation has been introduced into its amino acid sequence, are constructed, a gene expression cassette is constructed by joining a promoter and terminator capable of substantially functioning in yeast to each of those genes, the resulting gene expression cassette is further introduced into yeast and the resulting transformant is cultivated, a polyester can be produced and recovered from the culture in such a manner that very high productivity can be expected.

Thus, the present invention relates to use of a gene coding for a mutated Aeromonas caviae-derived PHA synthase in yeast.

Particularly, the present invention relates to a gene expression cassette
which comprises a gene coding for a mutated PHA synthase, and a promoter and a terminator both capable of functioning in yeast,
said gene coding for a mutated PHA synthase being obtainable by applying at least one of the following amino acid substitutions from (a) to (h) to an Aeromonas caviae-derived PHA synthase having the amino acid sequence shown under SEQ ID NO:1;
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

Moreover, the invention relates to
a gene,
which is obtainable by adding a DNA coding for a peroxisome-targeting signal to a gene coding for the mutated PHA synthase.

In a preferred embodiment thereof, the invention relates to the gene as mentioned above
wherein the peroxisome-targeting signal comprises the amino acid sequence shown under SEQ ID NO:2 or SEQ ID NO:3, and more preferably to the gene as mentioned above
wherein the peroxisome-targeting signal-encoding DNA has the nucleotide sequence shown under SEQ ID NO:4 or SEQ ID NO:5.

In another preferred embodiment, the invention relates to the gene as mentioned above
wherein at least one CTG codon in the gene coding for an Aeromonas caviae-derived PHA synthase has been modified into TTA, TTG, CTT, CTC or CTA.

The present invention also relates to
a gene expression cassette
which comprises the above gene, a promoter and a terminator both capable of functioning in yeast.

The present invention further relates to
a transformant
which is resulted from transformation of at least one gene expression cassette above-mentioned into yeast, and preferably relates to
the above-mentioned transformant,
wherein the yeast is Candida maltosa.

Furthermore, the invention relates to
a method for producing polyesters,
which comprises cultivating the above transformant and recovering the product polyester from the culture obtained,
and preferably relates to
the above-mentioned method,
wherein the polyester is a copolyester obtainable by copolymerization of 3-hydroxybutyric acid represented by the following formula (1): 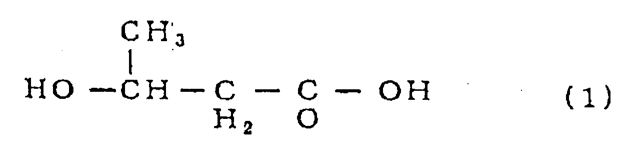 and 3-hydroxyhexanoic acid represented by the following formula (2):

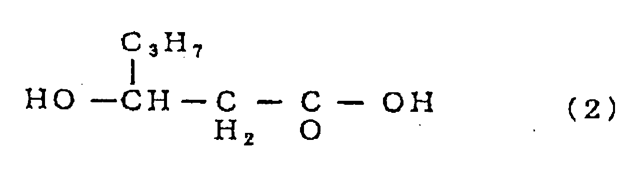

### DETAILED DESCRIPTION OF THE INVENTION

In the following, the present invention is described in detail.

### (1) A useful mutant of a polyester synthase

In the present invention, a mutated PHA synthase is used as a useful mutant, which is obtainable by applying at least one of the following amino acid substitutions from (a) to (h) to an Aeromonas caviae-derived PHA synthase having the amino acid sequence shown under SEQ ID NO:1;
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

In this description, for example, "Asn-149" means asparagine located at 149th position in the amino sequence, and amino acid substitution (a) means a conversion of asparagine located at 149th position into serine.

As a method to obtain the above mutant, there may be mentioned the following methods or the like. Various methods has been known to obtain modified mutant with improved properties such as enzyme activity, substrate specificity and/or thermal stability, by modifying amino acid sequences of polyester synthases derived from bacteria such as Aeromonas caviae. Especially, means utilizing molecular evolution technology (Japanese Kokai Publication 2002-199890) and the like are highly useful because desired mutants can be obtained in a short time. It is also possible to identify useful amino acid mutations on the basis of an enzyme conformation or an expectable conformation thereof by computing, for example, by using program Shrike (Japanese Kokai Publication 2001-184831) and the like.

It is not confirmed until actual applications whether thus-identified amino acid substitution (mutation) shows expected effects for the objective host or not. It is because the mutants of the above-mentioned T. Kichise et al. Appl. Environ. Microbiol. 68, 2411-2419 (2002) or Amara AA. et al., Appl. Microbiol. Biotechnol. 59, 477-482 (2002), which are obtained from Escherichia coli by means utilizing molecular evolution technology, are those most adaptable for the growing condition of Escherichia coli used for obtaining said mutants, and there is no guarantee that the mutants can adapt to different growing conditions, still less a different cell species. And it is also because mutants constructed by computing are not sufficient yet in accuracy.

Furthermore, combinations of mutations obtained by utilizing molecular evolution or the like technology makes it possible to give further suitable mutants. Therefore, mutants obtainable by applying at least two of the above amino acid substitutions from (a) to (h) are also favorably used.

### (2) Construction of mutated genes for yeast

As a gene coding for the mutant obtainable by the method described in (1), there may be mentioned, for example, a gene obtainable by applying a DNA mutation corresponding to the amino acid substitution mentioned in (1) to the gene coding for an Aeromonas caviae-derived PHA synthase having the amino acid sequence shown under SEQ ID NO:1, and the like genes. Preferable one is a gene obtainable by introducing a DNA mutation corresponding to the above amino acid substitution mentioned in (1) into a PHA synthase gene, which is adapted to yeast.

Such a PHA synthase gene, which is adapted to yeast, is not particularly restricted, but for example, there may be mentioned ORF2 (shown under SEQ ID NO:3 in WO 01/88144), which is a gene coding for, in Candida maltosa, the same amino acid sequence as the gene coding for an Aeromonas caviae-derived PHA synthase, and the like genes.

Namely, use of the bacterial gene as it is may lead to occurrence, in some host yeasts, of some abnormality in translation of genetic codes in some instances. For example, CTG codon is translated into serine, not into leucine, in Candida maltosa (H. Sugiyama et al., Yeast, 11, 43-52 (1995)). Such problem can be solved by using a modified gene wherein at least one CTG codon contained in the latter gene has been converted to another codon corresponding to leucine (TTA, TTG, CTT, CTC, or CTA) in advance. Furthermore, considering the frequency of codon usage composing a gene of the host in use, a gene, which has a modification converting a codon to another codon with high usage, may be constructed and utilized.

In introduction of the amino acid substitution (mutation) described in (1) into the above-mentioned PHA synthase gene, which is adapted to yeast, a mutation of a codon may be introduced into an objective site by using site-directed mutagenesis. The method to introduce a mutation into an amino acid sequence of a gene in a site-specific manner may be carried out by using the recombinant DNA method, the PCR method or the like. For example, when appropriate restriction sequences are present at the both sides of the objective site in the PHA synthase gene into which a mutation is introduced, it may be carried out by a cassette mutation method, which comprises cleaving the sequences by the corresponding restriction enzymes, removing the region containing the site into which an introduction of a mutation is required, and then inserting a DNA fragment which has a mutation introduced into only the objective site by chemical synthesis or the like. Introduction of a site-specific mutation by PCR may be carried out by amplifying one side of the PHA synthase gene with a primer for mutation, which has the objective mutation introduced into the site into which an introduction of the mutation is required, and a primer for amplification, which does not have a mutation comprising one terminal sequence of said gene, amplifying another side with a primer for mutation, which has a complementary sequence to the above primer for mutation, and a primer for amplification, which does not have a mutation comprising another terminal sequence of said gene, ,annealing the obtained two amplified fragments, and further carrying out PCR using the above two kinds of primers for amplification. The constructs obtained by a site-specific manner are confirmed by determination of the nucleotide sequences thereof. The determination of the nucleotide sequences may be carried out by a known manner in the art using an automatic nucleotide sequence analyzer or the like.

Generally, in fermentative production of a polyester using yeast, glucose, fats and oils, fatty acids and the like, which can be utilized by the yeast, can be used as carbon sources, without any particular restriction. Particularly, in fermentative polyester production using fats and oils, fatty acids, n-paraffins or the like as carbon sources, these carbon sources are metabolized via the β oxidation cycle, and the metabolic intermediates in the β oxidation cycle are utilized as substrates for polyester synthesis with good efficiency (T. Fukui, Y. Doi, J. Bacteriol., 179, No. 15, 4821-4830 (1997); Q. Ren et al., J. Bacteriol., 182, No. 10, 2978-2981 (2000)). Since the β oxidation in yeast is carried out in peroxisomes, which are intracellular microbodies, localization, in peroxisomes, of an enzyme involved in polyester synthesis is favorable for efficient polyester synthesis.

The proteins to be transferred to peroxisomes are synthesized on free ribosomes and, owing to the function of a peroxisome-targeting signal presenting in the protein sequences, they are transferred to peroxisomes (S. Subramani, J. Membrane Biol., 125, 99-106 (1992); Y. Itai, Kagaku to Seibutsu 35, No. 10, 687-695 (1997); E. H. Hettema, Biochim. Biophys. Acta, 1451, 17-34 (1999)).

Therefore, in the present invention, addition of a DNA coding for such peroxisome-targeting signal to a gene coding for an enzyme involved in polyester synthesis, namely the gene coding for a mutated PHA synthase, is preferable, and, by this addition, it becomes possible to localize the enzyme involved in polyester synthesis, namely a mutated PHA synthase, in peroxisomes for efficient polyester synthesis.

Known as peroxisome-targeting signals occurring at the carboxyl terminus are sequences comprising three amino acid residues, namely "(serine/alanine/cysteine)-(lysine/arginine/histidine)-leucine". The expression (serine/alanine/cysteine), for instance, as used herein means any one of serine, alanine and cysteine. For the targeting of the mutated PHA synthase toward peroxisomes, the addition of the above three-amino-acid sequence to the carboxyl terminus of the enzyme is sufficient. Among the peroxisome-targeting carboxyl-terminal signals, the most commonly known one, namely "serine-lysine-leucine" (hereinafter referred to as SKL for short) (SEQ ID NO:2) or "alanine-lysine-isoleucine" (hereinafter referred to as AKI for short) (SEQ ID NO:3), which is known as the peroxisome-targeting carboxyl-terminal signal in Candida tropicalis (J. D. Aitchison et al., J. Biol. Chem., 266, 23197-23203 (1991)), is preferably added to the carboxyl terminus.

The nucleotide sequence corresponding to such amino acid sequence is not particularly restricted. In the case of SKL, the nucleotide sequence shown under SEQ ID NO:4 can be utilized and, in the case of AKI, that shown under SEQ ID NO:5 can be used, for example.

Further, sequences occurring in the vicinity of the N terminus and comprising 9 amino acid residues, namely "(arginine/lysine)-(leucine/valine/isoleucine)-(5 amino acid residues)-(histidine/glutamine)-(leucine/alanine)", are also known as peroxisome-targeting signals. By inserting and adding these sequences into the amino acid sequence of.the mutated PHA synthase, it is possible to cause localization of the enzyme in peroxisomes.

In order to add a DNA coding for such a peroxisome-targeting signal as mentioned above to the gene coding for the mutated PHA synthase, chemical synthesis, the PCR method or the like method can be used.

### (3) Gene expression cassette construction

The gene expression cassette of the present invention comprises the mutated gene of (2), a promoter and a terminator both capable of functioning in yeast.

For the above mutated gene of (2) to be expressed in yeast, it is necessary that such DNA sequences as a promoter, the upstream activating sequence (UAS), etc. occur on the 5' upstream of the gene and such DNA sequences as poly(A) additional signal, terminator, etc. on the 3' downstream of the gene. When an appropriate site satisfying the above conditions occurs on a yeast chromosome, the gene in question may be directly inserted therein. Alternatively, the gene may be inserted into a plasmid having an appropriate promoter and terminator so that yeast may be transformed by the resulting plasmid.

In the practice of the present invention, a gene expression cassette is preferably constructed by ligating a promoter to the gene on the 5' upstream thereof and a terminator on the 3' downstream thereof so that the cassette may be used in transforming yeast.

Any promoter and terminator sequences may be used provided that they can function in yeast, which is to be a host. While, among the promoters, there are ones causing constitutive expression and ones causing inducible expression, either type of promoter may be used. In the practice of the present invention, it is desirable that, when Candida maltosa is used as a host, the promoter and terminator can function in Candida maltosa, hence the promoter and terminator be derived from Candida maltosa. More preferably, the Candida maltosa ALK1, ALK2 or ALK5 gene-derived promoter and the ALK1 gene-derived terminator are utilized.

Thus, for example, the promoter ALK1p (WO 01/88144) of the Candida maltosa ALK1 gene (GenBank D00481), the promoter ALK5p (SEQ ID NO:6) of the ALK5 gene, and the like, can be used as the promoter. Furthermore, a promoter (Kogure, et al., Abstract of the lectures of Japan Society for Bioscience, Biotechnology, and Agrochemistry, p. 191 (2002)), which is improved in its promoter activity by an addition of plural of ARR (alkane responsible region) sequences on the upstream of these promoters, can be utilized (SEQ ID NO:7). The terminator ALK1t (WO 01/88144) of the Candida maltosa ALK1 gene and the like terminator can be used as the terminator.

The nucleotide sequences of the above promoters and/or terminators each may be the nucleotide sequences in which one or a plurality of nucleotides may have undergone deletion, substitution and/or addition provided that they can function in Candida maltosa. In this description, the expression "the nucleotide sequences in which one or a plurality of nucleotides may have undergone deletion, substitution and/or addition" means the nucleotide sequences with deletion, substitution, and/or addition, wherein such number of nucleotides to be capable of being deleted, substituted and/or added by the known method in the prior art, such as "Protein-Nucleic acid-Enzyme, a special issue, gene-amplifying PCR method TAKKAJ 35(17), 2951-3178(1990)" or "PCR technology(1990), edited by Henry A. Erlich, translated by Ikunoshin Kato", etc. are deleted, substituted and/or added.

The promoter is ligated to the 5' upstream of the gene coding for the mutated Aeromonas caviae-derived PHA synthase with an added DNA coding for a peroxisome-targeting signal, and the terminator is ligated to the 3' downstream of the gene coding for the mutated PHA synthase with the added DNA coding for a peroxisome-targeting signal.

The vector to be used in construction of the gene expression cassette may be any of those capable of autonomous replication in Escherichia coli. It may further have a region capable of autonomous replication in yeast. The vector capable of autonomous replication in yeast is maintained in microbial cells. It is also possible to integrate the gene expression cassette into a chromosome.
As an example of such vector, pUTU1 capable of autonomous replication in Candida maltosa can be used (M. Ohkuma, et al., J. Biol. Chem., vol. 273, 3948-3953 (1998)).

The method of constructing the gene expression cassette according to the present invention by joining the promoter and terminator to the structural gene is not particularly restricted. Except for ones represented in the example section to be described below, of the present invention, the PCR method can be utilized in order to form appropriate restriction sites. The method described in WO 01/88144 can be used, for example.

### (4) Host

The "yeast" so referred to herein is not particularly restricted. Thus, usable are yeasts deposited with organism depositories (e.g. IFO, ATCC, etc.) and belonging to such genera as Aciculoconidium, Ambrosiozyma, Arthroascus, Arxiozyma, Ashbya, Babjevia, Bensingtonia, Botryoascus, Botryozyma, Brettanomyces, Bullera, Bulleromyces, Candida, Citeromyces, Clavispora, Cryptococcus, Cystofilobasidium, Debaryomyces, Dekkera, Dipodascopsis, Dipodascus, Eeniella, Endomycopsella, Eremascus, Eremothecium, Erythrobasidium, Fellomyces, Filobasidium, Galactomyces, Geotrichum, Guilliermondella, Hanseniaspora, Hansenula, Hasegawaea, Holtermannia, Hormoascus, Hyphopichia, Issatchenkia, Kloeckera, Kloeckeraspora, Kluyveromyces, Kondoa, Kuraishia, Kurtzmanomyces, Leucosporidium, Lipomyces, Lodderomyces, Malassezia, Metschnikowia, Mrakia, Myxozyma, Nadsonia, Nakazawaea, Nematospora, Ogataea, Oosporidium, Pachysolen, Phachytichospora, Phaffia, Pichia, Rhodosporidium, Rhodotorula, Saccharomyces, Saccharomycodes, Saccharomycopsis, Saitoella, Sakaguchia, Saturnospora, Schizoblastosporion, Schizosaccharomyces, Schwanniomyces, Sporidiobolus, Sporobolomyces, Sporopachydermia, Stephanoascus, Sterigmatomyces, Sterigmatosporidium, Symbiotaphrina, Sympodiomyces, Sympodiomycopsis, Torulaspora, Trichosporiella, Trichosporon, Trigonopsis, Tsuchiyaea, Udeniomyces, Waltomyces, Wickerhamia, Wickerhamiella, Williopsis, Yamadazyma, Yarrowia, Zygoascus, Zygosaccharomyces, Zygowilliopsis, and Zygozyma.

The yeast to be used as the transformant host in the practice of the present invention is not particularly restricted but, among them, preferably is one belonging to the genus Candida or Yarrowia, more preferably Candida maltosa or Yarrowia lipolytica, particularly preferably Candida maltosa.

Among the yeasts usable as the transformant hosts, the Candida maltosa AC16 strain has been internationally deposited with the National Institute of Advanced Industrial Science and Technology, Central 6, 1-1-1 Higashi, Tsukuba, Ibaraki, Japan, on November 15, 2000 under the accession number FERM BP-7366.

### (5) Transformant preparation

The transformant of the present invention is resulted from introduction of at least one gene expression cassette of (3) into yeast.

The gene expression cassette recombinant vector involved in polymer synthesis can be introduced into yeast in the conventional manner, using, for example, the calcium phosphate method (Lederberg, E. M. et al., J. Bacteriol., 119, 1072 (1974)), the electroporation method (Current Protocols in Molecular Biology, vol. 1, 1.8, p. 4 (1994)), or the like. Commercially available transformation kits such as Fast Track TM-Yeast Transformation Kit SM (Geno Technology) can also be utilized.

As an example, the Candida maltosa CHA1 strain (S. Kawai, et al., Agric. Biol. Chem., vol. 55, 59-65 (1991)) can be used as the host. By transforming this strain using a plasmid vector containing the gene expression cassette involved in polymer synthesis, and the like, by the transformation method mentioned above, it is possible to produce Candida maltosa transformants having a plasmid such as pARR-ORF2S, represented in the example section to be described below, or the like plasmid.

Each of the transformant AC16 pUTA-149NSx2 (originally deposited domestically on May 8, 2003 under the accession number FERM BP-10019, and then transferred to the Budapest Treaty depositry), which is transformed by the plasmid pARR-149NSx2, and the plasmid pARR-149NS/171DGx2 (deposited internationally on April 27, 2004 under the accession number FERM BP-10017 to the Budapest Treaty depositry) has been internationally deposited with the National Institute of Advanced Industrial Science and Technology, Central 6, 1-1-1 Higashi, Tsukuba, Ibaraki, Japan.

### (6) Polyester production

In accordance with the polyester production method of the present invention, a polyester is recovered from the culture obtainable by cultivating the above-mentioned transformant of the present invention.

Namely, the polyester production of the present invention can be carried out by adding the transformant of the present invention into a medium, culturing the same, and then recovering a polyester from the cultured cells or the culture obtained. The cultivation temperature is within a temperature range in which the organism can grow, preferably 15°C to 40°C, more preferably 20°C to 40°C, and still more preferably 28°C to 34°C. The cultivation time is not particularly restricted but, for example, 1 to 7 days are preferred in the case of a batch culture. Moreover, a continuous culture can be also utilized.

The medium is not particularly restricted provided that yeast can utilize. Media containing a carbon source, a nitrogen source, an inorganic salt, other organic nutrient sources and the like can be used, for example.

The carbon source is not particularly restricted provided that yeast can assimilate, carbohydrates, fats and oils, fatty acids, n-paraffins and the like can be used. As the carbohydrates, there may be mentioned, for example, glucose, sucrose and glycerol, etc. As the fats and oils, there may be mentioned, for example, rapeseed oil, coconut oil, palm oil and palm kernel oil, etc. As the fatty acids, there may be mentioned, for example, hexanoic acid, octanoic acid, decanoic acid, lauric acid, oleic acid, palmitic acid, linolic acid, linolenic acid, myristic acid, and like saturated and unsaturated acids, as well as esters and salts of these fatty acids and other fatty acid derivatives. As the n-paraffins, there may be mentioned, for example, dodecane and tetradecane, etc.

When the promoter expression is of the inducible type, an inducer (e.g. alcohols and the like) is to be added appropriately. In some instances, the inducer may serve as the main carbon source.

As an example, Candida maltosa can be cultivated using fats or oils as carbon sources. In the case of yeast, which cannot assimilate fats or oils efficiently or at all, improvements can be achieved by adding lipase to the medium. Furthermore, yeast can be provided with the ability to assimilate fats and oils by transformation with a lipase gene.

As the nitrogen source, there may be mentioned, for example, ammonia, ammonium chloride, di-ammonium sulfate, ammonium phosphate, and other ammonium salts, as well as peptone, meat extract, yeast extract, and the like.

As the inorganic salts, there may be mentioned, for example, potassium dihydrogenphosphate, di-potassium hydrogenphosphate, magnesium phosphate, magnesium sulfate, sodium chloride, and the like.

The other organic nutrient sources include, for example, amino acids such as glycine, alanine, serine, threonine, proline and the like; vitamins such as vitamin B1, vitamin B12, biotin, nicotinamide, pantothenic acid, vitamin C and the like; and the like.

In the practice of the present invention, the polyester is recovered from yeast cells using the following method, for instance. After completion of cultivation, cells are separated from the culture fluid using a centrifuge and the like and the cells are washed with distilled water and methanol or the like, and then dried. The polyester is extracted from these dried cells using an organic solvent such as chloroform and the like. The cell fraction is removed from the organic solvent solution containing the polyester by filtration and the like. A poor solvent, such as methanol, hexane or the like, is added to the filtrate to cause the polyester to precipitate out. The precipitate polyester is separated from the supernatant by filtration or centrifugation, and dried.
The polyester can be thus recovered. Since yeast cells are used as polyester producer cells in accordance with the present invention, such simple and easy methods of separation and recovery as mentioned above can be utilized.

The polyester obtained is analyzed by, for example, gas chromatography, nuclear magnetic resonance spectrometry and/or the like. Molecular weights can be determined by GPC method. Recovered dried polymers are dissolved in chloroform, and then this solution may be analyzed by Shimadzu GPC system equipped with Shodex K805L (product of Showa Denko) using chloroform as a mobile phase. Commercial standard polystyrene and the like may be used as the standard molecular weight sample.

As described above, in the present invention, by constructing a transformant of Candida maltosa using a gene expression cassette which comprises a gene coding for a mutated Aeromonas caviae-derived PHA synthase, which is improved in its enzymatic activity, or a gene resulting from addition of a DNA coding for a peroxisome-targeting signal to a gene coding for a mutated Aeromonas caviae-derived PHA synthase, which is improved in its enzymatic activity, together with a terminator and a promoter both capable of functioning in yeast, and then cultivating the same transformant, polyesters can be synthesized efficiently.

In the present invention, as polyesters, the copolyester P(3HB-co-3HH), which results from copolymerization of 3-hydroxybutyric acid represented by the general formula (1) and 3-hydroxyhexanoic acid represented by the general formula (2), can be preferably produced.

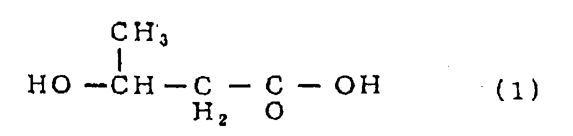

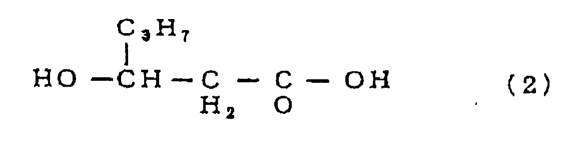

### BRIEF DESCRIPTION OF THE DRAWINGS

Fig. 1 is a schematic diagram of construction of pSTARR from pUAL1, and a simple diagram of each plasmid, as described in the example section.
Fig. 2 is a schematic diagram of construction of pARR-149NSx2 and the like from pSTARR, and a simple diagram of each plasmid, as described in the example section.

### BEST MODE FOR CARRYING OUT THE INVENTION

The following examples illustrate the present invention more specifically. These examples are, however, by no means limitative of the technical scope of the present invention.

### (Example 1) Synthesis and modification of PHA synthase gene

Based on the amino acid sequences of an Aeromonas caviae-derived PHA synthase (T. Fukui, et al., FEMS Microbiology Letters, vol. 170, 69-75 (1999)) shown under SEQ ID NO:1, the corresponding PHA synthase gene was synthesized.

Since Candida maltosa is yeast translating the CTG codon into serine, not leucine, CTG was not assigned to the leucine codon. That codon with high codon usage in Candida maltosa was preferentially selected as the codon corresponding to each amino acid. For codon usage, Klaus Wolf: Nonconventional Yeast in Biotechnology (published by Springer) was referred to. In this way, the PHA synthase gene (ORF2) (SEQ ID NO:3 in WO 01/88144) was designed and chemically synthesized. Then, the product was cloned into pUCNT (described in WO 94/03613).

Next, pUCNT, in which a PHA synthase gene was cloned, was amplified using SEQ ID NOs:8 and 9, as primers for PCR, for substitution of serine for asparagine, which is 149th amino acid of said PHA synthase. Pfu polymerase (product of Stratagene) was used for the amplification. After 18 cycles of PCR (each cycle comprising: 1 minute at 96°C, 1 minute at 60°C, 11 minutes at 68°C), a restriction enzyme DpnI was added to thereby cleave a template plasmid, then transforming E. coli JM109 strain, and recovering a plasmid from the resultant transformant. Using primers of SEQ ID NOs:10 to 14, the nucleotide sequence thereof was analyzed. The nucleotide sequence was determined by using DNA sequencer 310 Genetic Analyzer (product of PERKIN ELMER APPLIED BIOSYSTEMS). Thus, the gene coding for a mutated PHA synthase, named ORF2-149NS, in which gene mutation was introduced into an objective site, was constructed.

### (Example 2) Construction of an expression cassette comprising a gene for a mutated PHA synthase

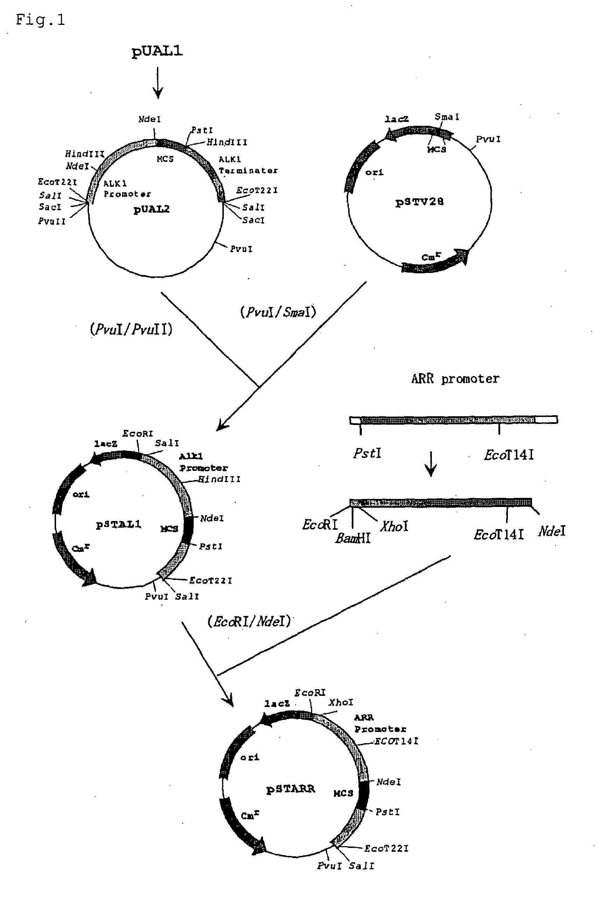

For causing expression of PHA synthase in Candida maltosa, it was decided that a Candida maltosa-derived promoter be ligated to the 5' upstream of each gene, and a Candida maltosa-derived terminator to the 3' downstream of each gene. It was decided to use the promoter ARRp, in which the ARR sequence was added to the upstream of Alk2 gene (GenBank X55881) promoter, as the promoter, and to ligate the Candida maltosa AlK1 gene (GenBank D00481) terminator ALK1t to the 3' downstream of each. ARRp gene imparted from Tokyo University (SEQ ID No:15) was converted, so as to be digested with XhoI and NdeI, by ligating EcoRI-XhoI linker to the PstI site and the synthetic DNA shown under SEQ ID No:16 to the EcoT14I site. The vector pUAL1 (described in WO 01/88144) was cleaved by EcoRI, then converted to be blunt-ended form, and subjected the resultant to ligation, to construct pUAL2, in which EcoRI-cleaved site was removed. The pUAL2 was digested with PvuI/PvuII, and ligated to the PvuI SmaI site of pSTV28 (Takara Shuzo) to construct pSTAL1. This pSTAL1 was digested with EcoRI/NdeI, and ligated to the above-mentioned ARRp to construct pSTARR. Fig. 1 shows a schematic diagram of construction of pSTARR from pUAL1 and a schematic diagram of each plasmid.

It was decided that a peroxisome-targeting signal be added to the carboxy terminus of each of ORF2 and ORF2-149NS mentioned in Example 1 so that each of them may be expressed in Candida maltosa and targeted to peroxisomes. The amino acid sequence of Ser-Lys-Leu (SKL) was used as the peroxisome-targeting signal to be added to the carboxy terminus. With the ORF2 and ORF2-149NS as templates, which are cloned in pUCNT, ORF2S and ORF2S-149NS were constructed using SEQ ID NOs:17 and 18 as primers. These genes coding for a mutated PHA synthase were ligated at NdeI and PstI sites of pSTARR, to thereby construct pSTARR-ORF2S and pSTARR-ORF2S149NS.

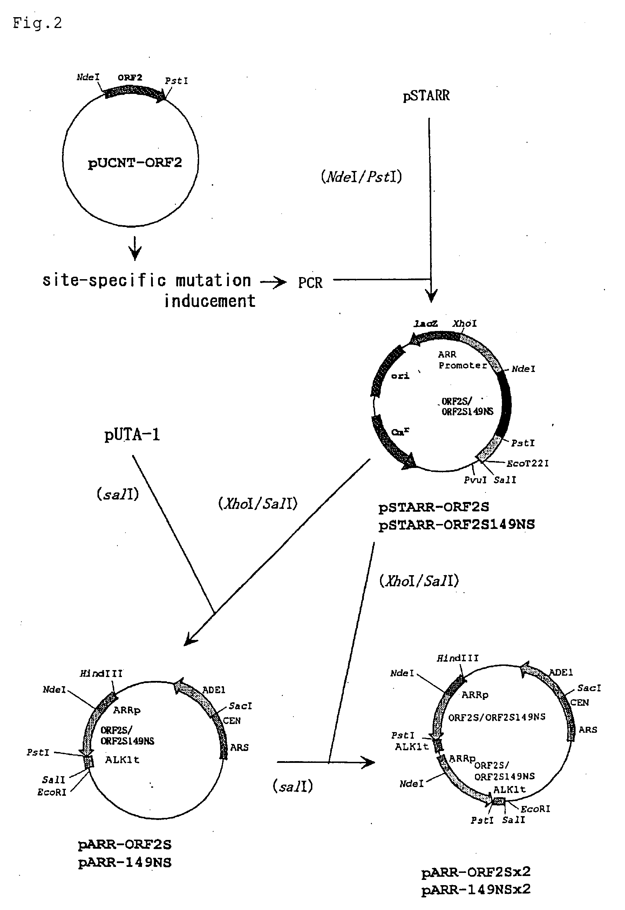

As a vector to finally ligate to the gene coding for a mutated PHA synthase, pUTA-1 (described in WO 01/88144) was used. An expression cassette was excised from pSTARR-ORF2S and pSTARR-ORF2S149NS with XhoI/SalI and ligated to SalI site of pUTA-1 to thereby construct pARR-ORF2S and pARR-149NS. Furthermore, the constructed pARR-ORF2S and pARR-149NS were digested with SalI, and expression cassettes, which were excised with XhoI/SalI from pSTARR-ORF2S and pSTARR-ORF2S-149NS, were ligated, to construct pARR-ORF2Sx2 and pARR-149NSx2. Fig. 2 shows a schematic diagram of construction of pARR-149NSx2 and the like from pSTARR and a simple diagram of each plasmid.

### (Example 3) Transformant construction

Unless otherwise specified, the reagents used in yeast cultivation were commercial products available from Wako Pure Chemical Industries. The host used was the Candida maltosa AC16 strain, which is a strain with the ADE1 gene disrupted and has been internationally deposited with the National Institute of Advanced Industrial Science and Technology International Patent Organism Depositary (accession number FERM BP-7366), and the plasmids comprising the above-mentioned gene expression cassettes of the present invention, namely pARR-ORF2S, pARR-149NS, pARR-ORF2Sx2, and pARR-149NSx2, were respectively introduced into the host. Each plasmid constructed was introduced into the host by the electroporation technique. The gene transfer apparatus used was ELECTRO CELL MANIPULATOR 600 (product of BTX). The cuvettes used were BM 6200 cuvettes produced by BIO MEDICAL CORPORATION CO. LTD. Each plasmid (1 µl) was added to 100 µl of competent cells. 100 µl of the thus-prepared competent cell/plasmid solution was taken and poured into a cuvette, which was set on the pulse generator. Then, electric pulses were applied under the following conditions: electrostatic capacity 40 µF, resistance value 246 ohm, and voltage 1.9 kV. After pulse application, 1 ml of 1 M sorbitol was added to each cuvette and, after gentle mixing, the cuvette was allowed to stand at room temperature for 1 hour. After plasmid introduction, the cells were cultured on a selection plate (0.67 w/v % Yeast Nitrogen base without amino acid (product of Difco), 2 w/v % glucose, 2 w/v % agar), to give transformants. Among them, the one harboring the plasmid pARR-149NSx2 has been internationally deposited as AC16 pUTA-149NSx2 with the National Institute of Advanced Industrial Science and Technology International Patent Organism Depositary (FERM BP-10019).

### (Example 4) Polymer production using the transformants

The Candida maltosa transformants resulting from introduction of the gene necessary for polymer production were cultivated in the following manner. YNB medium (0.67 w/v % Yeast Nitrogen base without amino acid, 2 w/v % Glucose) was used as a medium for preculture, and M2 medium (12.75 g/L ammonium sulfate, 1.56 g/L potassium dihydrogenphosphate, 0.33 g/L dipotassium hydrogenphosphate trihydrate, 0.08 g/L potassium chloride, 0.5 g/L sodium chloride, 0.41 g/L magnesium sulfate heptahydrate, 0.4 g/L calcium nitrate heptahydrate, and 0.01 g/L Iron(III) trichloride tetrahydrate) supplemented with 2 w/v % palm oil and 0.45 ml/L of trace elements dissolved in hydrochloric acid (1 g/mL Iron(II) sulfate heptahydrate, 8 g/mL zinc(II) sulfate heptahydrate, 6.4 g/mL manganese(II) sulfate tetrahydrate, and 0.8 g/mL cuprous(II) sulfate pentahydrate) was used as a production medium.

A 500-ml Sakaguchi flask containing 50 ml of the above medium for preculture was inoculated with 500 µl of a glycerol stock of each transformant and, after 20 hours of cultivation, the culture was inoculated into a 2-L Sakaguchi flask containing 300 mL of the production medium at an inoculum size of 10 v/v %. This was cultivated at an incubation temperature of 30°C and a shaking speed of 90 rpm for 2 days. Cells were recovered from the culture fluid by centrifugation, suspended in 80 ml of distilled water, and disrupted using an ultrahigh pressure homogenizer (APV's Rannie 2000, at 15,000 Psi for 15 minutes), followed by centrifugation. The precipitate obtained was washed with methanol and then lyophilized.

The lyophilized cells were ground, 100 ml of chloroform was added thereto, and the mixture was stirred overnight for effecting extraction. The cells were removed by filtration, the filtrate was concentrated to 1 to 2 ml using an evaporator, and about 10 ml of hexane was added to the concentrate to cause the polymer to precipitate out. The cultivation results thus obtained are shown in Table 1. The molecular weights were measured as follows. The recovered dried polymer (10 mg) was dissolved in 5 ml of chloroform, and then solid matters were removed therefrom by filtration. This obtained solution was analyzed by Shimadzu GPC system equipped with Shodex K805L (300 x 8 mm, two columns were connected)(product of Showa Denko) using chloroform as a mobile phase. As the standard molecular weight sample, commercial standard polystyrene was used and the molecular weights were determined as weight-average molecular weight. The 3HH molar fraction was measured with NMR (JOEL, JNM-EX400).

**Table 1**

| Plasmid | Polymer content (wt%) | 3HH molar fraction (mol%) | Molecular weight (M.W.) |
|---|---|---|---|
| pARR-ORF2S | 2.1 | 15.4 | 450000 |
| pARR-ORF2Sx2 | 2.97 | 21.7 | 260000 |
| pARR-149NS | 7.1 | 23.8 | 710000 |
| pARR-149NSx2 | 12. 8 | 28.4 | 455000 |

As shown in the above results, the yeast transformed by plasmids pARR-149NS and pARR-149NSx2 containing the expression cassette of the present invention, which comprises a mutated gene coding for a mutated PHA synthase, increased polymer content and 3HH molar fraction as compared with the case of the yeast transformed by plasmids pARR-ORF2S and pARR-ORF2Sx2 containing an expression cassette comprising a gene coding for a wild-type PHA synthase. From these results, not only improving effect in activities of the mutated PHA synthases of the present invention was demonstrated, but also tendency of increase of the obtained PHA polymers was found. Thus, usefulness of the mutated PHA synthase of the present invention was demonstrated.

### (Example 5) Double-mutant

A mutant, in which glycine was substituted for aspartic acid, 171st amino acid of the PHA synthase, was constructed according to the method described in Example 1. SEQ ID NOs:19 and 20 were used for the primers to introduce the mutation. In addition, a double-mutant, in which serine was substituted for 149th amino acid asparagine and glycine was substituted for 171st amino acid aspartic acid, was constructed by the same method as in Example 1, using ORF2-149NS, a gene coding for a mutated PHA synthase constructed in Example 1, as a template. Thus, ORF2-171DG, a gene coding for a mutated PHA synthase, and ORF2-149NS/171DG, a gene coding for a double-mutated PHA synthase, were completed.

Next, using these mutated genes, pSTARR-ORF2S171DG and pSTARR-ORF2S149NS/171DG were constructed according to the method described in Example 2, by adding a signal sequence using SEQ ID NOs:17 and 18 as primers, and cloning them into plasmid pSTARR. By introducing these mutated gene expression cassettes into pUTA-1 according to the method described in Example 2, plasmids pARR-171DG and pARR-149NS/171DG were constructed. Then, an expression cassette using the terminator LACt (SEQ ID NO:21) of LAC4 gene (GenBank M84410) in lieu of the terminator ALK1t was constructed. ALK1t in pSTARR-ORF2S171DG and pSTARR-ORF2S149NS/171DG were removed by PstI/SalI, and a plasmid, into which LAC4t amplified with primers shown under SEQ ID NOs:22 and 23 was introduced, was constructed instead.
This expression cassette comprising a gene coding for a mutated PHA synthase, in which the terminator was substituted, was excised by XhoI/SalI, respectively, and then ligated to SalI site of plasmids pARR-171DG and pARR-149NS/171DG, to complete plasmids pARR-171DGx2 and pARR-149NS/171DGx2, into which two expression cassettes comprising a gene coding for a mutated PHA synthase were introduced. Among them, the plasmid pARR-149NS/171DGx2 has been internationally deposited with the National Institute of Advanced Industrial Science and Technology International Patent Organism Depositary (FERM BP-10017).

These plasmids were introduced into Candida Maltosa AC16 strain (FERM BP-7366) according to the method described in Example 3, and polymer production and analysis were carried out according to the method described in Example 4. The results are shown in Table 2, and were compared with that of Example 4.

**Table 2**

| Plasmid | Polymer content (wt%) | 3HH molar fraction (mol%) | Molecular weight (M. W.) |
|---|---|---|---|
| pARR-ORF2Sx2 | 2.97 | 21.7 | 260000 |
| pARR-149NSx2 | 12.8 | 28.4 | 455000 |
| pARR-171DGx2 | 4. 5 | 23.0 | 330000 |
| pARR-149NS/171DGx2 | 14.0 | 34.1 | 320000 |

As shown in the above results, the yeast transformed by plasmids pARR-171DGx2 containing the expression cassette of the present invention, which comprises a mutated gene coding for a mutated PHA synthase, increased polymer content as compared with the case of using the yeast transformed by plasmid pARR-ORF2Sx2 containing an expression cassette comprising a gene coding for a wild-type PHA synthase. Furthermore, it was revealed that the yeast transformed with the plasmid pARR-149NS/171DGx2 containing an expression cassette comprising a double-mutated gene coding for a double-mutated PHA synthase further increased polymer content as well as 3HH molar fraction. Thus, usefulness of the mutated PHA synthase of the present invention was demonstrated.

### INDUSTRIAL APPLICABILITY

The present invention made it possible to produce copolyesters resulting from copolymerization of a 3-hydroxyalkanoic acid(s) such as 3-hydroxybutyric acid and 3-hydroxyhexanoic acid each represented by the general formula (1) and (2) and having biodegradability and good physical properties in yeast efficiently.

## Claims

1. A gene expression cassette
which comprises a gene coding for a mutated polyhydroxyalkanoic acid synthase, and a promoter and a terminator both capable of functioning in yeast,
said gene coding for a mutated polyhydroxyalkanoic acid synthase being obtainable by applying at least one of the following amino acid substitutions from (a) to (h) to an Aeromonas caviae-derived polyhydroxyalkanoic acid synthase having the amino acid sequence shown under SEQ ID NO:1;
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

2. A gene,
which is obtainable by adding a DNA coding for a peroxisome-targeting signal to a gene coding for a mutated polyhydroxyalkanoic acid synthase,
said gene being obtainable by applying at least one of the following amino acid substitutions from (a) to (h) to an Aeromonas caviae-derived polyhydroxyalkanoic acid synthase having the amino acid sequence shown under SEQ ID NO:1;
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

3. The gene according to Claim 2,
wherein the peroxisome-targeting signal comprises the amino acid sequence shown under SEQ ID NO:2 or SEQ ID NO:3.

4. The gene according to Claim 3,
wherein the peroxisome-targeting signal-encoding DNA has the nucleotide sequence shown under SEQ ID NO:4 or SEQ ID NO:5.

5. The gene according to Claim 2 to 4,
wherein at least one CTG codon in the gene coding for an Aeromonas caviae-derived polyhydroxyalkanoic acid synthase has been modified into TTA, TTG, CTT, CTC or CTA.

6. A gene expression cassette
which comprises the gene according to any of Claims 2 to 5, a promoter and a terminator both capable of functioning in yeast.

7. A transformant
which is resulted from introduction of at least one gene expression cassette according to Claim 1 or 6 into yeast.

8. The transformant according to Claim 7,
wherein the yeast is Candida maltosa.

9. A method of producing polyesters,
which comprises cultivating the transformant according to Claim 7 or 8 and recovering the product polyester from the culture obtained.

10. The method according to Claim 9,
wherein the polyester is a copolyester obtainable by copolymerization of 3-hydroxybutyric acid represented by the following formula (1): 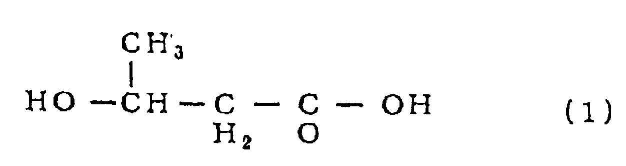 and 3-hydroxyhexanoic acid represented by the following formula (2):


SEQUENCE LISTING

<110> KANEKA CORPORATION

<120> IMPROVED TRANSFORMANT AND PROCESS FOR PRODUCING POLYSESTER USING THE SAME

<130> B030172WO01

<150> JP 2003-137492
<151> 2003-5-15

<160> 23

<170> PatentIn Ver. 2.1

<210> 1
<211> 594
<212> PRT
<213> Aeromonas caviae

<400> 1
Met Ser Gln Pro Ser Tyr Gly Pro Leu Phe Glu Ala Leu Ala His Tyr
 1               5                  10                  15

Asn Asp Lys Leu Leu Ala Met Ala Lys Ala Gln Thr Glu Arg Thr Ala
            20                  25                  30

Gln Ala Leu Leu Gln Thr Asn Leu Asp Asp Leu Gly Gln Val Leu Glu
        35                  40                  45

Gln Gly Ser Gln Gln Pro Trp Gln Leu Ile Gln Ala Gln Met Asn Trp
    50                  55                  60

Trp Gln Asp Gln Leu Lys Leu Met Gln His Thr Leu Leu Lys Ser Ala
65                  70                  75                  80

Gly Gln Pro Ser Glu Pro Val Ile Thr Pro Glu Arg Ser Asp Arg Arg
                85                  90                  95

Phe Lys Ala Glu Ala Trp Ser Glu Gln Pro Ile Tyr Asp Tyr Leu Lys
            100                 105                 110

Gln Ser Tyr Leu Leu Thr Ala Arg His Leu Leu Ala Ser Val Asp Ala
        115                 120                 125

Leu Glu Gly Val Pro Gln Lys Ser Arg Glu Arg Leu Arg Phe Phe Thr
    130                 135                 140

Arg Gln Tyr Val Asn Ala Met Ala Pro Ser Asn Phe Leu Ala Thr Asn
145                 150                 155                 160

Pro Glu Leu Leu Lys Leu Thr Leu Glu Ser Asp Gly Gln Asn Leu Val
                165                 170                 175

Arg Gly Leu Ala Leu Leu Ala Glu Asp Leu Glu Arg Ser Ala Asp Gln
            180                 185                 190

Leu Asn Ile Arg Leu Thr Asp Glu Ser Ala Phe Glu Leu Gly Arg Asp

        195                 200                 205

Leu Ala Leu Thr Pro Gly Arg Val Val Gln Arg Thr Glu Leu Tyr Glu
    210                 215                 220

Leu Ile Gln Tyr Ser Pro Thr Thr Glu Thr Val Gly Lys Thr Pro Val
225                 230                 235                 240

Leu Ile Val Pro Pro Phe Ile Asn Lys Tyr Tyr Ile Met Asp Met Arg
                245                 250                 255

Pro Gln Asn Ser Leu Val Ala Trp Leu Val Ala Gln Gly Gln Thr Val
            260                 265                 270

Phe Met Ile Ser Trp Arg Asn Pro Gly Val Ala Gln Ala Gln Ile Asp
        275                 280                 285

Leu Asp Asp Tyr Val Val Asp Gly Val Ile Ala Ala Leu Asp Gly Val
    290                 295                 300

Glu Ala Ala Thr Gly Glu Arg Glu Val His Gly Ile Gly Tyr Cys Ile
305                 310                 315                 320

Gly Gly Thr Ala Leu Ser Leu Ala Met Gly Trp Leu Ala Ala Arg Arg
                325                 330                 335

Gln Lys Gln Arg Val Arg Thr Ala Thr Leu Phe Thr Thr Leu Leu Asp
            340                 345                 350

Phe Ser Gln Pro Gly Glu Leu Gly Ile Phe Ile His Glu Pro Ile Ile
        355                 360                 365

Ala Ala Leu Glu Ala Gln Asn Glu Ala Lys Gly Ile Met Asp Gly Arg
    370                 375                 380

Gln Leu Ala Val Ser Phe Ser Leu Leu Arg Glu Asn Ser Leu Tyr Trp
385                 390                 395                 400

Asn Tyr Tyr Ile Asp Ser Tyr Leu Lys Gly Gln Ser Pro Val Ala Phe
                405                 410                 415

Asp Leu Leu His Trp Asn Ser Asp Ser Thr Asn Val Ala Gly Lys Thr
            420                 425                 430

His Asn Ser Leu Leu Arg Arg Leu Tyr Leu Glu Asn Gln Leu Val Lys
        435                 440                 445

Gly Glu Leu Lys Ile Arg Asn Thr Arg Ile Asp Leu Gly Lys Val Lys
    450                 455                 460

Thr Pro Val Leu Leu Val Ser Ala Val Asp Asp His Ile Ala Leu Trp
465                 470                 475                 480

Gln Gly Thr Trp Gln Gly Met Lys Leu Phe Gly Gly Glu Gln Arg Phe
                485                 490                 495

Leu Leu Ala Glu Ser Gly His Ile Ala Gly Ile Ile Asn Pro Pro Ala
            500                 505                 510

Ala Asn Lys Tyr Gly Phe Trp His Asn Gly Ala Glu Ala Glu Ser Pro
        515                 520                 525

Glu Ser Trp Leu Ala Gly Ala Thr His Gln Gly Gly Ser Trp Trp Pro
    530                 535                 540

Glu Met Met Gly Phe Ile Gln Asn Arg Asp Glu Gly Ser Glu Pro Val
545                 550                 555                 560

Pro Ala Arg Val Pro Glu Glu Gly Leu Ala Pro Ala Pro Gly His Tyr
                565                 570                 575

Val Lys Val Arg Leu Asn Pro Val Phe Ala Cys Pro Thr Glu Glu Asp
            580                 585                 590

Ala Ala

<210> 2
<211> 3
<212> PRT
<213> Artificial Sequence

<220>
<223> Peroxisome Targetting Sequence

<400> 2
Ser Lys Leu

<210> 3
<211> 3
<212> PRT
<213> Artificial Sequence

<220>
<223> Peroxisome Targetting Sequence

<400> 3
Ala Lys Ile

<210> 4
<211> 9
<212> DNA
<213> Artificial Sequence

<220>
<223> Peroxisome Targetting Sequence

<400> 4
tctaaattg

<210> 5
<211> 9
<212> DNA

<213> Artificial Sequence

<220>
<223> Peroxisome Targetting Sequence

<400> 5
gctaaaatt                                                              9

<210> 6
<211> 750
<212> DNA
<213> Candida maltosa

<220>
<223> ALK5 Promoter

<400> 6
aagcttcaca tggatcaatt gcgtttgtca catgtggtca tccagctatg gttgatgagg 60
ttagatattt tacttgtaag aatattaaca acccagaaaa gaaaagagtt gatttctttg 120
aacaagtgca agtctgggct tagacgttta tttttgtttt tgttgagtgg taatacatat 180
tcttcgtatc tatgaagatt tttcacacgc ggatagtaat tgtactagcc gcttctttaa 240
gtaactgatt tacccaacaa gtacatggta atacaaactc tcactcacta gacttcgctt 300
ctagttgctt caaattagac ggttataatg tatgccaagg ttttgtgtaa tttcacggtg 360
attaaccttt tccccttttt atactcctca ttatccacga tgtaatctga tctatgaacg 420
tgataagtaa cattacttag tcattaagta tggccaattc agttatacat attagtaatg 480
ctccacatcc attgtattca tatgtaatgc caaatatcac attcatttac acagaatcgg 540
ttttgttaaa tactccgcta ttgtacagca acaataggat tatgtacaga atgaaaaaca 600
aaaggcggag aaattcgacg gaaaaattta ttatttacaa atcgtattcc cgcattatct 660

ataaaacaga ttcaaaataa tctagatctc ttttttttgc ttccttttat ttctttttaa 720

ataagattaa actaaaaata tgattgatga 750

<210> 7

<211> 73

<212> DNA

<213> Candida maltosa

<220>

<223> ARR

<400> 7
   catgtgcttt tttttttgtt ttcaatttga aagttttttt atttccgcaa tacaaaatta 60
   ttttttatcc gct 73

<210> 8

<211> 32

<212> DNA

<213> Artificial Sequence

<220>

<223> Primer

<400> 8

gatggagcca tagcggagac gtattgtcta gt 32

<210> 9

<211> 32

<212> DNA

<213> Artificial Sequence

<220>

<223> Primer

<400> 9

actagacaat acgtctccgc tatggctcca tc 32

<210> 10

<211> 22

<212> DNA

<213> Artificial Sequence

<220>

<223> Primer

<400> 10

actaacttgg atgatttggg tc 22

<210> 11

<211> 22

<212> DNA

<213> Artificial Sequence

<220>
<223> Primer

<400> 11
ctaatttctt ggctactaac cc 22

<210> 12
<211> 22
<212> DNA
<213> Artificial Sequence

<220>
<223> Primer

<400> 12
agttttgatc gttccaccat tc 22

<210> 13
<211> 20
<212> DNA
<213> Artificial Sequence

<220>
<223> Primer

<400> 13
catgaaccaa ttatcgccgc 20

<210> 14

<211> 22

<212> DNA

<213> Artificial Sequence

<220>

<223> Primer

<400> 14

gttggtttct gccgttgatg at                                                22

<210> 15

<211> 754

<212> DNA

<213> Artificial Sequence

<220>

<223> ARR-Alk2 promoter

<400> 15

aagcttgcat gcctgcaggt cgaaattcga gctcggtacc cggggatcct ctagagtcca 60

tgtgcttttt tttttgtttt caatttgaaa gtttttttat ttccgcaata caaaattatt 120

ttttatccgc tcatgtgctt ttttttttgt tttcaatttg aaagtttttt tatttccgca 180

atacaaaatt attttttatc cgctgaccca gatcctctag agtccatgtg cttttttttt 240

tgttttcaat ttgaaagttt ttttatttcc gcaatacaaa attatttttt atccgctcat 300

gtgctttttt ttttgttttc aatttgaaag tttttttatt tccgcaatac aaaattattt 360

tttatccgct gacccagatc ctctagagtc catgtgcttt tttttttgtt ttcaatttga 420
aagttttttt atttccgcaa tacaaaatta ttttttatcc gctcatgtgc tttttttttt 480
gttttcaatt tgaaagtttt tttatttccg caatacaaaa ttatttttta tccgctgacc 540
cagatctcga ctctagagga tccccgtttt tttatttccg caatacaaaa ttatttttta 600
tccgctttcc gttcctttct tcttgtgata aatctcaaca attatatata tcattccata 660
accctgaata attttttttt taagtccttg gtttcttttt ttagaaaaaa aggtgaatca 720
gtaaaatttt tgttatttat cattttaact caca 754

<210> 16
<211> 83
<212> DNA
<213> Artificial Sequence

<220>
<223> Synthetic DNA

<400> 16
gtccttggtt tcttttttta gaaaaaaagg tgaatcagta aaatttttgt tatttatcat 60
tttaactcac atatgaagat atc 83

<210> 17
<211> 45
<212> DNA
<213> Artificial Sequence

<220>
<223> primer

<400> 17
atggtactgc agttacaatt tagaagcagc atcttcttcg gttgg                45

<210> 18
<211> 99
<212> DNA
<213> Artificial Sequence

<220>
<223> Primer

<400> 18
acacatatgt ctcaaccatc ttatggtcca ttgttcgaag ctttggctca ttacaatgat 60
aaattgttgg ctatggctaa agctcaaacc gaaagaact                       99

<210> 19
<211> 29
<212> DNA
<213> Artificial Sequence

<220>
<223> Primer

<400> 19
gactttggaa tccggtggtc aaaatttgg                                  29

<210> 20

<211> 29

<212> DNA

<213> Artificial Sequence

<220>

<223> Primer

<400> 20

ccaaattttg accaccggat tccaaagtc                                          29

<210> 21

<211> 340

<212> DNA

<213> Kluyveromyces lactis

<220>

<223> LACt

<400> 21

gaattttata cttagataag tatgtactta caggtatatt tctatgagat actgatgtat      60

acatgcatga taatatttaa acggttatta gtgccgattg tcttgtgcga taatgacgtt     120

cctatcaaag caatacactt accacctatt acatgggcca agaaaatatt ttcgaacttg     180

tttagaatat tagcacagag tatatgatga tatccgttag attatgcatg attcattcct     240

acaacttttt cgtagcataa ggattaatta cttggatgcc aataaaaaaa aaaaacatcg     300

agaaaatttc agcatgctca gaaacaattg cagtgtatca                           340

<210> 22

<211> 40

<212> DNA

<213> Artificial Sequence

<220>

<223> primer

<400> 22

gcgcctgcag gaattttata cttagataag tatgtactta 40

<210> 23

<211> 40

<212> DNA

<213> Artificial Sequence

<220>

<223> primer

<400> 23

ggcgtcgaca agcttgtttc tgagcatgct gaaattttct 40